# EUROPEAN PATENT APPLICATION

(11) **EP 2 388 020 A1**
(43) Date of publication of application: **23.11.2011**
(21) Application number: 09838423.3
(22) Date of filing: 02.02.2009
(51) Int. Cl.: A61L 27/14

(54) **CONCRETE SCAFFOLD MADE OF BONE POWDER AND FIBRIN GLUE**

(30) Priority: 16.01.2009 KR 20090003884
(71) Applicant: Industry-Academic Cooperation Foundation Wonkwang University, Jellabuk-do 570-749 (KR)
(72) Inventor: LEE, Jun, Daejeon 305-755 (KR); YOON, Dong-Hyeon, Gumi-si Gyeongsangbuk-do 730-040 (KR)
(74) Representative: Lecca, Patricia S.
(86) International application number: PCT/KR2009/000507
(87) International publication number: WO 2010/082702

(57) **Abstract**

The present invention relates to a bone-regenerating scaffold, and more particularly, to a bone-regenerating scaffold which is made of a mixture of fibrin glue and bone powder, the interior of which has a plurality of pores for accommodating bone growth factors, and which has a predetermined concrete shape. The present invention also relates to a method for manufacturing the scaffold

## Description

### [Technical Field]

The present invention relates to a scaffold for bone regeneration, and more particularly, to a scaffold for bone regeneration, which includes fibrin glue; bone powder mixed with the fibrin glue; and a plurality of pores formed to accommodate a bone growth promoting factor, wherein the scaffold has a predetermined concrete shape, and a method of preparing the scaffold.

### [Background Art]

Bone tissue engineering aims to induce bone regeneration by grafting engineeringly-cultured osteogenic cells onto a site requiring a bone and develop osseous tissues. This tissue engineering requires osteogenic cells, a scaffold to which the osteogenic cells are attached to survive, and a growth factor configured to promote bone induction and differentiation for bone regeneration. The respective components may form an osseous tissue when a stimulus is given to the components while adequate time and environments are maintained. Accordingly, there is much research to find an ideal factor for each component.

In particular, an osteoconductive material such as a scaffold configured to form a bone is important to allow osteogenic cells to live in. The osteoconductive material is similar to that in mineralization of bone, should be biocompatible, and provides surface reactivity and physical support closely associated with the surroundings of the bone. Various osteoconductive materials such as ceramic, collagen, a biodegradable polymer, etc have been studied.

Takahashi et al. reported synostosis using porous hydroxyapatite (HA) and bone morphogenic protein (BMP) (Takahashi K et al. J Biomed Master Res A12:117-123, 2007), Asahina et al. reported the use of HA, collagen, bovine BMP, etc. (Asahina I et al. J Med dent Sci. 44:63-69, 1997), Ohgushi et al. reported the use of a ceramic scaffold (Ohgushi H et al. J Biomed Mat Res 26: 885-95, 1992; Ohgushi H et al. J Biomed Mat Res 32: 341-8, 1996), and Caplan et al. reported the use of porous calcium phosphate, etc. (Caplan AI. J Cell Physiol 213(2): 341-347, 2007).

Moreover, European Patent No. 1231947 discloses a bone substitute material comprising (a) a soft matrix consisting of fibrin or fibrinogen, (b) a living cell, and (c) a fixed matrix consisting of non-ceramic hydroxyapatite cement.

Furthermore, Japanese Patent Publication No. 1999-513590 proposes a biodegradable, porous 3-dimensional (3D) bone grafting matrix including calcium phosphate particulates formed from mineralized collagen and having a particle size of 5 µm or less, the calcium phosphate particulates being fixed in a matrix.

In addition, US Patent Publication No. 2008-0213229 discloses a method of producing a fibrin contained semi-solid osteoblast composition to treat bone fracture, the method including isolating osteoblasts from a bone tissue of an animal, culturing/proliferating the isolated osteoblasts in Dulbecco's Modified Eagle's Medium (DMEM) or Minimum Essential Medium, Alpha Modification (α-MEM) to prepare an osteoblast suspension, and mixing the resulting osteoblast suspension with a semi-solid osteoblast coagulation factor.

However, conventional scaffolds do not function as media in which living osteoblasts can grow. Moreover, when the scaffolds are grafted in a bone defect area, their shapes and intensities are not maintained and they are not stably held in the area during treatment which leads to poor bone regeneration efficiency.

### [Disclosure]

### [Technical Problem]

The present inventor has made a great effort to develop a scaffold which can overcome the above-described problems, and found that a solid scaffold obtained by mixing fibrin glue and bone powder and freeze-drying the resulting mixture is stable in vivo, maintains a space in which cells can be continuously placed during bone reconstruction, and maintains its adequate shape and intensity even in an in vivo environment, thereby inducing rapid bone regeneration. Therefore, the present invention has been completed based on these facts. Moreover, by a freeze-drying process followed by mixing the fibrin glue and the bone powder, a large number of pores are formed in the mixture, thereby improving absorption and maintenance of a bone regeneration promoting factor.

Furthermore, the scaffold of the present invention may be freeze-dried in a cast having a predetermined desired shape to have a desired shape. This advantage makes it possible to provide a scaffold suitable for use in a bone defect area. In connection with the latest 3D scanning technology, it is possible to exactly determine a bone defect area in a patient and regenerate a shape of the scaffold, and thus the use of the present invention can be improved.

Therefore, an object of the present invention is to provide a solid scaffold for bone regeneration, in which bone powder is mixed with fibrin glue including fibrinogen and thrombin as main components.

More particularly, another object of the present invention is to provide a concrete scaffold for bone regeneration, in which a plurality of pores are formed to accommodate a bone growth promoting factor by mixing bone powder with fibrin glue including fibrinogen and thrombin as main components and freeze-drying the resultant mixture.

Still another object of the present invention is to provide a scaffold having a desired shape, which is obtained by putting the mixture of the fibrin glue and the bone powder into a cast having a predetermined shape and freeze-drying the mixture.

Yet another object of the present invention provides a patient-tailored scaffold which is obtained by preparing a cast corresponding to a bone defect area of a patient using a 3D digital scanning technique, putting the mixture of fibrin glue and bone powder into the cast and freeze-drying the mixture.

### [Technical Solution]

In order to accomplish the above objects, an aspect of the present invention provides a concrete scaffold for bone regeneration, in which bone powder is mixed with fibrin glue including fibrinogen and thrombin as main components. That is, the scaffold of the present invention is **characterized in that** the bone powder is added to the fibrin glue.

Moreover, the scaffold according to the present invention may be in a solid and concrete state. For this purpose, the fibrin glue and the bone powder may be mixed together, and the resultant mixture may be freeze-dried. Also, the scaffold according to the present invention may be treated to have a predetermined shape before being freeze-dried, or may be freeze-dried in a predetermined cast. In one aspect, the shape and the cast may correspond to a jawbone or tooth defect area of a patient. More particularly, the cast may be prepared by (a) preparing a 3D mold using 3D CT and (b) preparing a cast for preparation of a scaffold suitable for the bone defect area in the 3D mold using a dental resin.

In the present invention, the term "bone powder" refers to a ground bone powder, preferably a ground bone (inorganic) powder, from which osteoblasts are removed. The bone powder may be derived from at least one selected from the group consisting of an autogenous bone, an allogeneic bone, a xenogeneic bone, and a synthetic bone (for example, hydroxyapatite). In the present invention, the bone powder is commercially available from, for example, Dynagraft (Austem Co. Ltd.), Biocera (Oscotec Inc.), Bio-Oss (Jungsan Biomed Co. Ltd.), ICB (Purgo), MBCP (Purgo), etc.

In the present invention, the term "fibrin glue" refers to a biocompatible and biodegradable product including fibrinogen and thrombin as main components. The fibrin glue has been used in a variety of applications. For example, the fibrin glue has been clinically applied for substitution or reinforcement of sutures by applying fibrinogen, thrombin, calcium chloride, or a fibrinolytic enzyme inhibitor as a tissue adhesive to suture peripheral nerves and fine blood vessels through tissue agglutination of fibrin in Europe. Moreover, in Japan, the fibrin glue has been used as a surgical adhesive for the cerebral nerve surgery including a vascular surgery field, the orthopedic surgery such as bone adhesion, and arrest of bleeding in patients suffering from lacerated wound, etc. For example, Greenplast (Green Cross Corp.), Beriplast-P (Aventis), Tisseel (Baxter), etc. are commercially available.

The fibrin glue according to the present invention preferably includes fibrinogen and thrombin. The fibrinogen may be used in a concentration of 10 to 1000 mg/ml, and preferably 10 to 100 mg/ml. The thrombin may be used in a concentration of 0.1 to 1000 IU/ml, and preferably 1 to 100 IU/ml.

The fibrin glue according to the present invention may further include aprotinin or calcium chloride. Moreover, the fibrin glue according to the present invention may further include a water-soluble binder. The water-soluble binder may be a cell culture medium, distilled water, or blood.

In the present invention, when bone powder and fibrin glue are mixed together, an increase in the amount of the fibrin glue may increase the possibility of causing the toxicity, and therefore it is preferred to suitably adjust the content of the fibrin glue. Moreover, when the size of the scaffold is larger, it is preferred to increase the content of the bone powder to maintain the intensity and shape of the scaffold. In view of these facts, the fibrin glue and the bone powder may be mixed in a volume ratio of 1:1 to 10, preferably 1:1 to 5, and more preferably 1:1 to 3 in the present invention.

The bone growth promoting factor according to the present invention includes a variety of factors for promoting bone growth. For example, the bone growth promoting factor may include a hormone, a cytokine, a stem cell, etc. More particularly, the bone growth promoting factor may be a platelet-derived growth factor (PDGF) or a vascular endothelial growth factor (VEGF).

Since a large number of pores are formed in the mixture of fibrin glue and bone powder while the mixture is subjected to a freeze-drying process, the scaffold according to the present invention may improve absorption and maintenance of the bone growth promoting factor. The freeze-dried scaffold readily absorbs a medium containing the bone growth promoting factor and transfers the medium into the pores.

Another aspect of the present invention relates to a method of preparing a solid scaffold having a predetermined shape, the method including mixing bone powder with fibrin glue and freeze-drying the resultant mixture in a cast having a predetermined shape.

The freeze-drying process may be performed in a conventional manner, which means that the residual moisture is present in an amount of 2% w/w or less during the freeze-drying process. Moreover, the freeze-drying process according to the present invention may be performed using a commercially available freeze-dryer.

Still another aspect of the present invention relates to a method of preparing a patient-tailored scaffold suitable for a bone defect area of a patient to promote bone regeneration by applying the scaffold to the bone defect area. In particular, the method of preparing the patient-tailored scaffold according to the present invention includes (a) preparing a 3D mold using 3D CT, (b) preparing a cast for preparation of a scaffold suitable for a bone defect area in the 3D mold using a dental resin, and (c) putting a mixture of fibrin glue and bone powder into the cast and freeze-drying the mixture.

### [Advantageous Effects]

The solid scaffold for bone regeneration according to the present invention is stable in vivo and continuously improves the absorption and maintenance of a bone growth promoting factor such as a cell or a cytokine during bone regeneration. Moreover, the solid scaffold of the present invention may induce bone regeneration into an adequate shape since the solid scaffold has a shape suitable for the bone defect area, maintain a space in which various materials for bone regeneration can be placed, and maintains its adequate shape and intensity even in an in vivo environment, thereby inducing rapid bone regeneration.

### [Description of Drawings]

FIG. 1 shows a configuration of a fibrin glue kit used in the present invention.
FIG. 2 shows a freeze-dried Biocera/fibrin block according to the present invention.
FIG. 3 shows a process of preparing a freeze-dried Biocera/fibrin block according to the present invention (A: Mixture of MBCP and fibrin glue, B: Gel state before freeze-drying process, C: Freeze-drying process, D: Solid state after freeze-drying process).
FIG. 4A shows a mixture of MBCP and fibrin glue and FIG. 4B shows a state of an MBCP/fibrin block obtained by freeze-drying the mixture.
FIG. 5 shows the induction of anesthesia of a miniature pig as an experimental animal, the extraction of teeth and the extracted teeth, respectively.
FIG. 6 shows the positions in which a bone graft material according to the present invention is grafted in an alveolus defect area of a miniature pig.
FIG. 7 shows a process of grafting a bone graft material according to the present invention (A: Exposure of a grafted area by flap reflection of a residual alveolar ridge in a lower jaw, B: Formation of only a bone defect area in the form of a saddle, and C: Grafting of a mixture of MBCP and fibrin glue or a MBCP/fibrin block).
FIGS. 8A to 8D show bone density analysis using a Dicom viewer program (FIG. 8A: Adjustment of lines, FIG. 8B: Adjustment of thickness, FIG. 8C: Measurement of density, and FIG. 8D: Measurement of density divided by 3 fragments (10x10 pixel size, 3 mm thickness)).
FIG. 9 shows the results of 3D computer tomography with respect to the areas in which a bone graft material according to the present invention is grafted.
FIG. 10 shows the results of immunohistochemical staining with respect to the areas in which a bone graft material according to the present invention is grafted.

### [Mode for Invention]

Hereinafter, preferred embodiments of the present invention will be described with reference to the accompanying drawings.

Hereinafter, exemplary embodiments of the present invention will be described in detail below with reference to the accompanying drawings such that those skilled in the art to which the present invention pertains can easily practice the present invention.

### Example 1: Preparation of Scaffold of the invention

### 1-1. Preparation of Fibrin Glue

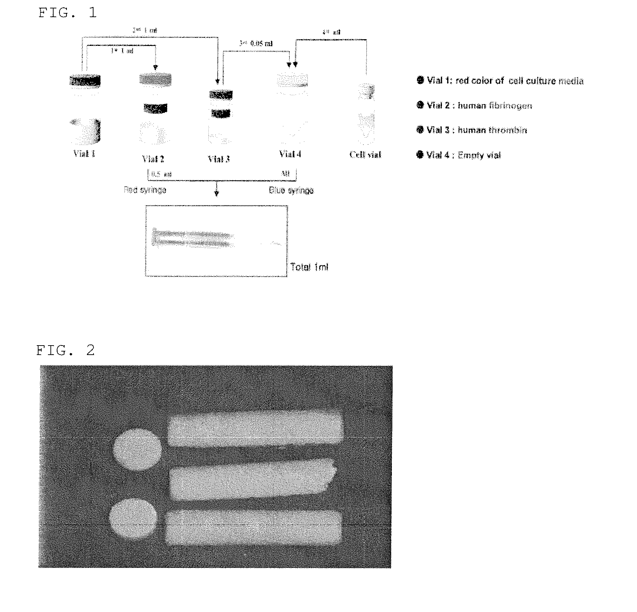

Fibrin glue was prepared using Greenplast kit® (Greenplast kit, Green Cross Corp., Korea) (FIG. 1). The preparation of the fibrin glue was performed as follows.

### 1) Preparation of Vial-1 solution: 5 ml

First, the protein concentration in vial-1 was determined as 97.6 mg/ml. In order to adjust the protein concentration 40 mg/ml, the solution was diluted 2.5 times. A 2.5 ml vial-2 solution was added to one vial-1 to dissolve the protein, and 2.5 ml of a physiological saline solution was added to another vial-1 to dissolve the protein. Then, the two completely dissolved vial-1 solutions were collected in one test tube.

### 2) Preparation of Vial-3 solution

The thrombin titer in vial-3 was determined as 557 IU/ml. In order to adjust the thrombin titer to 20 IU/ml, the solution was diluted 28 times. A 2.5ml vial-4 solution was added to one vial-3 to dissolve the protein, and 1 ml of the vial-4 solution was taken and added to 10 ml of a physiological saline solution, and mixed thoroughly.

### 1-2. Mixing of Fibrin Glue and Bone Powder

### 1) Disk-Type Bone Block

0.25 g of bone powder (Biocera) was weighed and put on each hole of a 24 hole plate. A 0.2 ml vial-1 solution was put into each hole and mixed with bone powder so that the bone powder could be sufficiently soaked in the vial-1 solution. Then, a 0.1 ml vial-3 solution was put into each hole and rapidly mixed using a spatula, and the resultant mixture was kept stationary.

### 2) Semicircular Rod-Type Bone Block

A prepared cast was used with a 10 ml pipette. 1.25 g of bone powder (Biocera) was weighed and put on each lane. A 2 ml vial-1 solution was put into each hole and mixed so that the bone powder could be sufficiently soaked in the vial-1 solution. A 0.5 ml vial-3 solution was put into each hole and rapidly mixed using a spatula, and the resultant mixture was kept at room temperature for one hour.

### 1-3. Freeze-Drying

The temperature of a shelf of a freeze-drier was set to -45°C and the freeze-drier was operated. As a result, a freeze-dried semicircular rod-type bone block was obtained (FIG. 2).

### Example 2: Materials and Methods for Experiments

### 2-1. Experimental Animals and Materials

Four 30 kg male miniature pigs (PWG, Korea) were used as experimental animals. The pigs were bred with soft animal feed and water at room temperature in certified breeding facilities for a predetermined period of time.

### 1) Tooth Extraction

Teeth from a premolar tooth to a first molar tooth were extracted from the left and right lower jaws of a miniature pig. After the extraction of the teeth, the wounds were continuously sutured. One week after the suturing process, an injection was performed, and the wounds were healed for one month. During the healing process, additional extraction of impacted teeth was performed during surgery.

### 2) Preparation of MBCP/Fibrin Block

### (1) Preparation of Fibrin Glue

Fibrin glue was prepared using a Greenplast kit® (Greenplst kit, Green Cross Corp., Korea). The 1 ml Greenplast kit includes concentrated human fibrinogen, aprotinin, human serum thrombin, and α-MEM (a medium solution). The aprotinin was mixed with the concentrated human fibrinogen to prepare a fibrinogen solution, and the α-MEM was mixed with the human serum thrombin to prepare a thrombin solution.

### (2) Preparation of MBCP/Fibrin Complex (hereinafter referred to as "MBCP+fibrin glue")

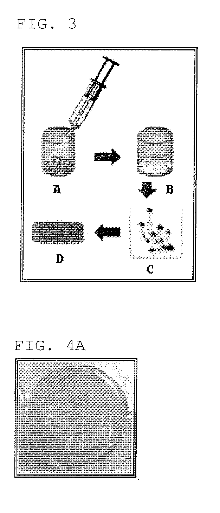

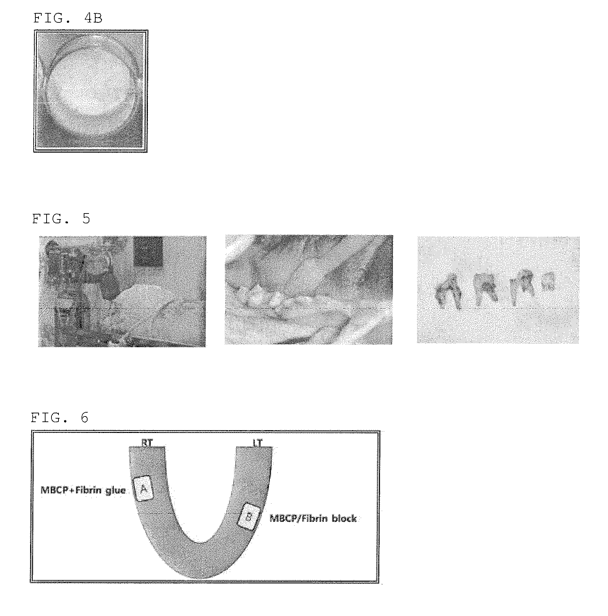

The previously prepared fibrin glue (containing the fibrinogen solution and the thrombin solution) was mixed with MBCP in a volume ratio of 1:1 and the resultant mixture was filled in a previously prepared cast. Then, the mixture was subjected to a freeze-drying process to prepare a MBCP/fibrin block (i.e., a scaffold of the present invention) (FIGS. 3 and 4).

### 2-2. Experimental Methods

### 1) Anesthesia Induction

For bone grafting of a miniature pig, each of an animal anesthetic (Rompun® 3 mg/kg, Bayer Korea Co., Ltd., Korea) and ketamine was intravenously injected to the miniature pig to induce general anesthesia. Oral intubation was performed to induce general anesthesia using N20 + 02.

In order to provide a field of vision, dogteeth of the upper and lower jaws were tied with a bandage to induce a maximum opening degree. The oral cavity was sterilized with Potadine, and 2% lidocaine (Yuhan Co, Ltd., Korea) containing epinephrine in a volume ratio of 1:100,000 was injected into the residual alveolar bone of the lower jaw to induce local anesthesia and stop bleeding (FIG. 5).

### 2) Alveolar Bone Exposure

The residual alveolar bone of the miniature pig was subjected to horizontal incision and vertical incision, and the periosteum was peeled off to expose the buccal and lingual sides of the lower jaw to the maximum.

### 3) Formation of Control and Experimental groups

Saddle type 2 wall-bony defects with a depth of 4 to 5 mm, a length of 8 mm, and a base width of 6 to 7 mm were formed using a micro-saw having a radius of 3 mm. Rough regions were polished into a desired shape using a surgical round bur or chisel or a mallet.

First, MBCP serving as a control was mixed with fibrin glue, and the resultant mixture was coagulated and filled in a right-hand bone defect area. Then, a MBCP/fibrin block serving as an experimental group was filled in the front of a left-hand bone defect (FIG. 6).

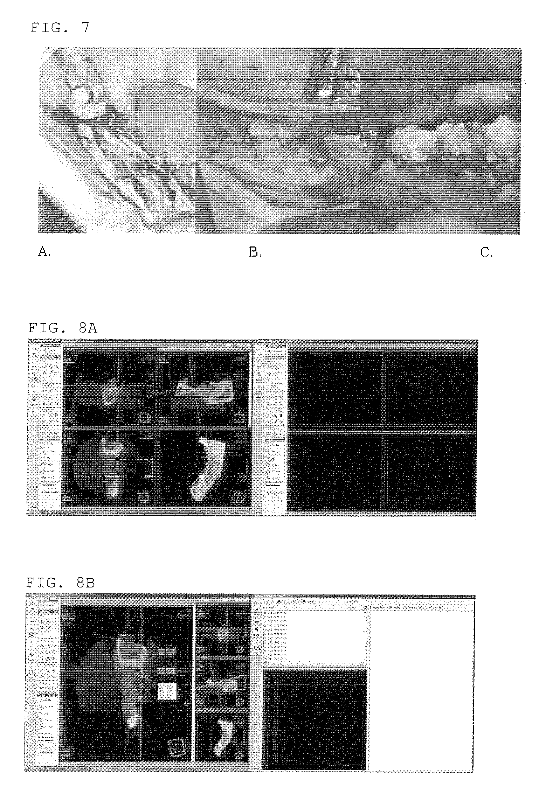

Releasing incision was performed on the wound to release the tension, and a 4-0 nylon was used to perform a continuous locking suture. Amoxicillin (Tiramox®, SAMJIN Pharmaceutical Co., Ltd., Korea) and diclofenac sodium (Kinpoin®, SAMJIN Pharmaceutical Co., Ltd., Korea) were intramuscularly injected into all experimental animals for three days to prevent infections after surgery, and the experimental animals were fed a soft diet with high-protein milk for one week. Disinfection was performed using chlorhexidine (Hexamedin®, Bukwang Pharmaceutical Co., Ltd., Korea) for one week (FIG. 7).

### 2-3. Visual Inspection

After surgery, disinfection of feeds provided to miniature pigs was continuously performed, and the miniature pigs were maintained under healthy conditions. 2, 4 and 8 weeks after the surgery, the miniature pigs were sacrificed, and the healing level of the bone graft area, the inflammatory condition, and the wound dehiscence were observed with the naked eye before the bone tissue samples were immobilized with formalin.

### 2-4. Computer Tomography Inspection

Subsequently, each bone graft area was examined using a standard digital radiograph and subjected to dental computer tomography. Afterwards, vertical and horizontal bone cuttings between bone graft areas were performed with respect to the center of the bone graft areas.

### 1) 3D Computer Tomography

The lower jawbone of the miniature pig containing each graft material was imaged using a Cone-beam CT (Alphard vega, Asahi Roentgen Ind. Co., Japan). The imaging was performed at a tube voltage of 80 KVp and a tube current of 8 mA for 15 seconds. Spatial resolutions of all specimens were set to 0.2 mm x 0.2 mm, and approximately 512 images per specimen were obtained. The obtained images were stored in ".dcm" formats, and these files were analyzed using a DICOM Viewer program (OnDemand 3 application, Cybermed, Korea), and all the images were identified and analyzed on a diagnostic monitor (WIDE, Korea).

### 2) Bone Density Analysis

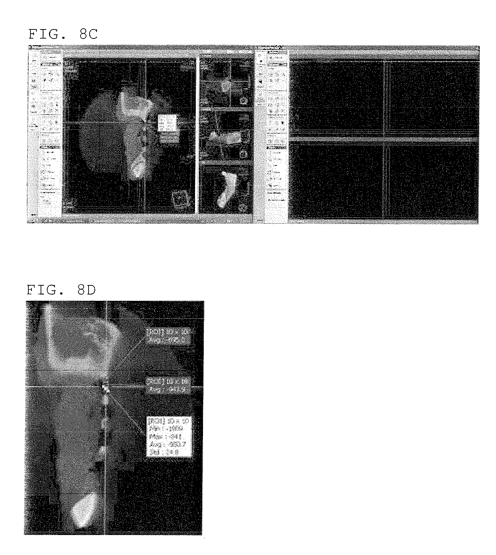

The axes of the coronal plane, the sagittal plane, and the transverse plane were adjusted with respect to the center of the bone defect area on the Dicom viewer program. The radiographic opacity of five regions such as center, up, down, left and right regions of the bone defect area on the transverse plane were measured with respect to the region of interest (ROI) of 10 pixel x 10 pixel. Three regions for each bone defect area such as front, middle and rear regions were measured, and the same analysis was performed on each miniature pig for 2, 4 and 8 weeks. Then, the radiographic opacity was statistically analyzed over time. Moreover, a change in radiographic opacity between experimental groups over time was statistically analyzed based on the analysis results. Here, the numerical values on this program were relative values representing the opacities ranging from - 1023 to 3071, and the cortical bone and the cancellous bone of the miniature pig were measured at a radiographic opacity of approximately 500 to 1300 and -100 to 600, respectively (FIG. 8).

### 2-5. Histological Inspection

Miniature pigs were sacrificed 2, 4 and 8 weeks after the surgery to obtain respective tissue samples. Then, the tissue samples were fixed in a 10 % neutral formalin solution for two days, demineralized with 10 % EDTA, and dehydrated and embedded in resin by a typical method. Then, 4 to 6 µm-sized microtomed samples were attached to a poly-L-lysine-coated slide to prepare samples. Tissue sections were prepared so that both the bone graft area and the normal area were included in the tissue sections, In order to observe the shapes of the new bone and fibrous tissue and examine the changes in shapes of the new bone and the fibrous tissue, the samples were stained with Hematoxylin & Eosin and Masson's trichrome stains and examined under a microscope.

### Example 3: Experimental Results

### 3-1. Clinical Features

Each of three miniature pigs in each group was analyzed at time points of 2, 4 and 8 weeks. It can be seen from the results of clinical evaluation that wound dehiscence appeared together with severe inflammation in the control at a time point of 2 weeks. However, in the other experimental groups, neither inflammation nor wound dehiscence appeared at time points of 4 and 8 weeks of the experiment, and excellent bone regeneration was observed (Table 1).

**[Table 1] Inflammation by MBCP+Fibrin Glue and MBCP/Fibrin Block**

| | **Bone graft material** | **2 Weeks** | **4 Weeks** | **8 Weeks** |
|---|---|---|---|---|
| **Group Control** | **MBCP+Fibrin glue** | **±** | **-** | **-** |
| Experimental group | MBCP/Fibrin block | - | - | - |

| | | | | |
|---|---|---|---|---|
| (-: negative, ±: rare, +: mild, ++: moderate, and +++: intense) | | | | |

### 3-2. Radiological Features

### 1) 3D Computer Tomography Evaluation

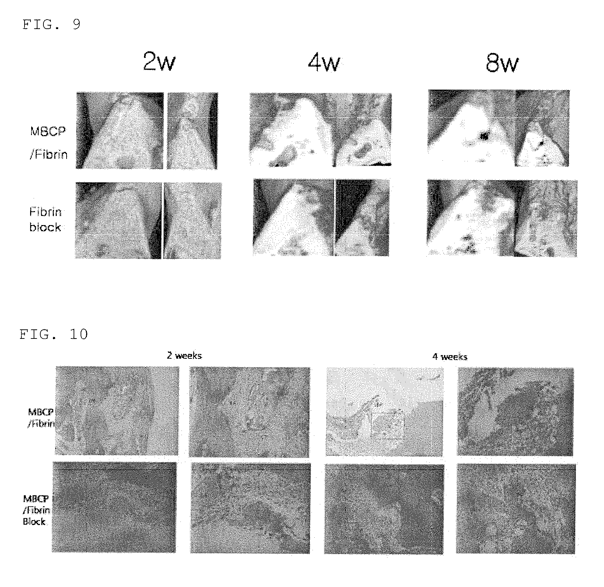

At a time point of 4 weeks, the MBCP+fibrin glue of the control was slightly absorbed into the buccal side and the lingual side while the height with respect to the overall shapes was slightly reduced. Moreover, the normal width of the alveolar material was in a range of approximately 2 to 3 mm, but the bone defect in the buccal side slightly appeared in the experimental group (MBCP/fibrin block) and the alveolar material showed a higher and wider shape (FIG. 9).

### 2) Bone Density Evaluation

In the radiological features, the bone density was increased in both the control and the experimental group during a normal ossification process. Here, the bone density was significantly increased in the experimental group, compared to the control. A higher bone density was observed especially at time points of 2 and 8 weeks in the freeze-dried MBCP/fibrin block group, compared to the control. This indicates that the initial graft particles were stabilized by blocking the synthetic bone and fibrin at a time point of 2 weeks, and the bone maturation by premature ossification was observed at a time point of 8 weeks (Table 2).

**[Table 2]**

| **Group** | **Bone graft material** | **2 Weeks** (*) | **4 Weeks** (*) | **8 Weeks** (*) |
|---|---|---|---|---|
| Control | MBCP+Fibrin glue | 120.67 (± 5.62) | 183.30 (± 4.4) | 187.90 (± 4.67) |
| Experimental group | MBCP/Fibrin block(*) | *161.17 (± 5.31) | 190.90 (± 2.00) | *234.07 (± 7.18) |

| | | | | |
|---|---|---|---|---|
| * p < 0.05 | | | | |

### 3-3. Histological Features

As a result, in the control in which MBCP was mixed with the fibrin glue and coagulated, clinical features such as osteoclast activities and peripheral angiogenesis around the grafted bone were observed at a time point of 2 weeks, which led to the observation of partial new bone regeneration. Moreover, the bone remodeling was further progressed at a time point of 4 weeks, and the regeneration of new bones was further increased. In the experimental group in which the MBCP/fibrin block was grafted into the bone defect area, the MBCP was dispersed in the fibrin structure at a time point of 2 weeks, but new blood vessels appeared while the structure of fibrin was partially destroyed. Furthermore, the absorption of the dispersed MBCP and the regeneration of new bones around the grafted bone were observed. In addition, the osteoblast activities were observed more clearly at a time point of 4 weeks, and thus the periphery of the bone defect area was replaced with a mature bone (FIG. 10).

In conclusion, saddle-type 2-wall bone defect areas were formed in the lower jawbone of the miniature pig, and the MBCP/fibrin mixture or the freeze-dried MBCP/fibrin block was grafted into the bone defect area to compare the osteogenic effects visually, radiologically and histologically at times points of 2, 4 and 8 weeks after the surgery, and the following comparison results were obtained.
1. In the visual inspection and 3D tomographic inspection, excellent bone regeneration was observed and good bone-grafted shape was maintained in the MBCP/fibrin block-grafted group, compared to the MBCP/fibrin glue-grafted group.
2. In the radiological bone density inspection, a higher bone density was measured in the MBCP/fibrin block-grafted group, compared to the MBCP/fibrin glue-grafted group, which showed that the bone regeneration was improved.
3. In the histological inspection, the new bone regeneration was excellent and the bone remodeling progressed more rapidly in the MBCP/fibrin block-grafted group, compared to the MBCP/fibrin glue-grafted group.

### [Industrial Applicability]

As described above, when the freeze-dried MBCP/fibrin block is grafted onto the bone defect area, it has a good shape-maintaining property and the bone regeneration is highly improved, compared to the conventional scaffold (MBCP/fibrin glue).

## Claims

1. A scaffold for bone regeneration comprising:
fibrin glue;
bone powder mixed with the fibrin glue; and
a plurality of pores formed to accommodate a bone growth promoting factor,
wherein the scaffold has a predetermined concrete shape.

2. The scaffold according to claim 1, wherein the scaffold is treated to have a predetermined shape before being freeze-dried.

3. The scaffold according to claim 1, wherein the scaffold is freeze-dried in a predetermined cast.

4. The scaffold according to claim 3, wherein the cast is prepared by (a) preparing a 3-dimensional (3D) skull mold using 3D computed tomography (CT) and (b) preparing a cast for preparation of the scaffold suitable for a bone defect area using a dental resin in the 3D skull mold.

5. The scaffold according to claim 1, wherein the bone powder is a ground bone powder from which osteoblasts are removed.

6. The scaffold according to claim 5, wherein the bone powder is derived from at least one selected from the group consisting of an autogenous bone, an allogeneic bone, a xenogeneic bone, and a synthetic bone.

7. The scaffold according to claim 1, wherein the fibrin glue comprises fibrinogen and thrombin.

8. The scaffold according to claim 7, wherein the fibrinogen is present in a concentration of 10 to 1000 mg/ml.

9. The scaffold according to claim 7, wherein the thrombin is present in a concentration of 0.1 to 1000 IU/ml.

10. The scaffold according to claim 1, wherein the fibrin glue further comprises aprotinin or calcium chloride.

11. The scaffold according to claim 1, wherein the fibrin glue further comprises a water-soluble binder.

12. The scaffold according to claim 11, wherein the water-soluble binder is a cell culture medium, distilled water, or blood.

13. The scaffold according to claim 1, wherein the bone powder and the fibrin glue are mixed in a volume ratio of 1 to 10:1.

14. The scaffold according to claim 1, wherein the bone growth promoting factor is a hormone, a cytokine, or a stem cell.

15. A method of preparing a scaffold for bone regeneration, the method comprising:
mixing bone powder and fibrin glue; and
freeze-drying the resultant mixture,
wherein the scaffold comprises a plurality of pores formed to accommodate a bone growth promoting factor and has a predetermined concrete shape.

16. The method according to claim 15, wherein the scaffold is treated to have a predetermined shape before being freeze-dried.

17. The method according to claim 15, wherein the freeze-drying process is performed in a predetermined cast.

18. The method according to claim 17, wherein the cast is prepared by (a) preparing a 3D skull mold using 3D CT and (b) preparing a cast for preparation of the scaffold suitable for a bone defect area using a dental resin in the 3D skull mold.

19. The method according to claim 15, wherein the bone powder is a ground bone powder from which osteoblasts are removed.

20. The method according to claim 19, wherein the bone powder is derived from at least one selected from the group consisting of an autogenous bone, an allogeneic bone, a xenogeneic bone, and a synthetic bone.

21. The method according to claim 15, wherein the fibrin glue comprises fibrinogen and thrombin.

22. The method according to claim 21, wherein the fibrinogen is present in a concentration of 10 to 1000 mg/ml.

23. The method according to claim 21, wherein the thrombin is present in a concentration of 0.1 to 1000 IU/ml.

24. The method according to claim 15, wherein the fibrin glue further comprises aprotinin or calcium chloride.

25. The method according to claim 15, wherein the fibrin glue further comprises a water-soluble binder.

26. The method according to claim 25, wherein the water-soluble binder is a culture medium, distilled water, or blood.

27. The method according to claim 15, wherein the bone powder and the fibrin glue are mixed in a volume ratio of 1 to 10:1.

28. The method according to claim 15, wherein the bone growth promoting factor is a hormone, a cytokine, or a stem cell.
